# EUROPEAN PATENT APPLICATION

(11) **EP 4 772 213 A2**
(43) Date of publication of application: **08.07.2026**
(21) Application number: 26177028.3
(22) Date of filing: 08.10.2021
(51) Int. Cl.: A61M 5/20

(54) **AUTOINJECTOR NEEDLE-HOUSING ASSEMBLY**

(30) Priority: 17.11.2020 US 202063114554 P; 15.12.2020 EP 20214349
(62) Divisional of application: 21790430.9
(71) Applicant: SHL Medical AG, 6302 Zug (CH)
(72) Inventor: BENDEK, Antonio, Deerfield Beach, FL 33442 (US)

(57) **Abstract**

The invention concerns an autoinjector needle-housing assembly (10) extending along an axis (12) in a longitudinal direction (13) from a proximal end (14) to a distal end (16), the autoinjector needle-housing assembly (10) comprising a housing (20) comprising a proximal part (25) and a distal part (30) attached to the proximal part (25), a needle (40) attached to the proximal part (25) of the housing (20), and a sterility barrier (50) attached to the distal part (30) of the housing (20), wherein the housing (20) and the sterility barrier (50) form a first sealed cavity (55), wherein a distal end (44) of the needle (40) is in the first sealed cavity (55), wherein the autoinjector needle-housing assembly (10) comprises a recess (82) formed by the distal end of the autoinjector needle-housing assembly (10), wherein the recess (82) is shaped to receive a cartridge (70) and to form a second sealed cavity (80) between the autoinjector needle-housing assembly (10) and said cartridge (70). Corresponding methods are also described.

## Description

### TECHNICAL FIELD

The invention concerns needle-housing assemblies, and particularly autoinjector needle-housing assemblies comprising a sterile cavity.

### BACKGROUND

An injection needle assembly is described in WO2014/076225 in which sealing in between components of a needle assembly is addressed. Whilst this can provide a sterile environment around the needle, the applicant has appreciated that further improvements can be made to this design to improve the sterile environment.

EP2450072 describes a system for dispensing one medicament followed by a primary medicament. WO2015/185664 describes an activating mechanism for a medicament delivery device. WO2017089270 describes a medicament injection device configured to receive a medicament cartridge sealed by a first penetrable barrier.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component.

Similarly, the terms "radial", "transverse", "transversal" and "transversally" refer to a direction generally perpendicular to the longitudinal direction, and "circumferential" is the direction around a longitudinal axis of the device and/or component.

A first aspect of the invention concerns an autoinjector needle-housing assembly extending along an axis in a longitudinal direction from a proximal end to a distal end, the autoinjector needle-housing assembly comprising a housing comprising a proximal part and a distal part attached to the proximal part, a needle fixedly attached to the proximal part of the housing, and a sterility barrier attached to the distal part of the housing, wherein the housing and the sterility barrier form a first sealed cavity, wherein a distal end of the needle is in the first sealed cavity, wherein the autoinjector needle-housing assembly comprises a recess formed by the distal end of the autoinjector needle-housing assembly, wherein the recess is shaped to receive a cartridge and to form a second sealed cavity between the autoinjector needle-housing assembly and said cartridge. The sterility barrier can be a separate component from the housing or an integral part of the housing. Preferably, the distal end of the needle is spaced apart from the sterility barrier.

This autoinjector needle-housing assembly can provide a complete sterile environment, not just for the needle but also for the part of the cartridge that the needle pierces. This can reduce the chance of contaminants.

Preferably, the recess is formed between the sterility barrier of the autoinjector needle-housing assembly and said cartridge. Alternatively, the recess is formed between the sterility barrier, the distal part of the housing and the cartridge.

Preferably, the sterility barrier comprises a tubular portion and a wall extending across the tubular portion. Preferably, the tubular portion of the sterility barrier extends in the longitudinal direction and the wall extends in a radial direction relative to the axis. Preferably, the wall extends across a proximal part of the tubular direction.

Preferably, the sterility barrier comprises a seal on the tubular portion of the sterility barrier. The seal can be on the inner surface of the tubular portion or the outer surface of the tubular portion. A seal on the inner surface seal can help seal the join with the cartridge, and a seal on the outer surface can help seal the join with the housing. Preferably, the seal is an O-ring. In general, providing seals can allow for greater manufacturing tolerances. Providing seals can also allow for lower friction when the sterility barrier is moving relative to the cartridge, which can be advantageous as it can reduce the force required to initiate an injection.

Preferably, the distal part of the housing comprises a tubular section and the proximal part of the housing comprises a wall extending in a radial direction relative to the longitudinal axis. Preferably, the wall extends across the proximal end of the tubular section.

Preferably, the first sealed cavity is tubular, and is formed by the tubular section of the housing, the wall of the housing and the wall of the sterility barrier.

Preferably, the tubular section of the sterility barrier is in the tubular section of the housing. Preferably, the sterility barrier is movable in the longitudinal direction relative to the housing.

Preferably, the needle is fixed relative to the housing. The needle preferably extends in the longitudinal direction from a proximal end to a distal end, and one or both of the proximal end and the distal end comprise angled tips. This can help to pierce the injection site and the cartridge (typically the cartridge septum) respectively.

A second aspect of the invention concerns an autoinjector sub-assembly comprising the autoinjector needle-housing assembly of any previous claim and a needle shield (preferably a rigid needle shield) attached to the autoinjector needle-housing assembly. Preferably, the needle shield and the autoinjector needle-housing assembly (preferably the wall of the housing of the autoinjector needle-housing assembly) define a third sealed cavity. Preferably, a proximal end of the needle is in the third sealed cavity.

A third aspect of the invention concerns an autoinjector comprising a cartridge and either a autoinjector needle-housing assembly as described above or the autoinjector sub-assembly as described above, wherein a proximal end of the cartridge is in the distal recess of the autoinjector needle-housing assembly. Preferably, the autoinjector comprises a cartridge holder extending around the cartridge.

A fourth aspect of the invention concerns a method of assembly of an autoinjector or an autoinjector sub-assembly, the method comprising the steps of: providing an autoinjector needle-housing assembly extending along an axis in a longitudinal direction from a proximal end to a distal end, the autoinjector needle-housing assembly comprising a housing comprising a proximal part and a distal part attached to the proximal part, a needle attached to the proximal part of the housing, and a sterility barrier attached to the distal part of the housing, wherein the housing and the sterility barrier form a first sealed cavity, wherein a distal end of the needle is in the first sealed cavity, wherein the autoinjector needle-housing assembly comprises a recess formed by the distal end of the autoinjector needle-housing assembly, wherein the recess is shaped to receive a cartridge and to form a second sealed cavity between the autoinjector needle-housing assembly and said cartridge; and inserting a cartridge into the recess to create a second sealed cavity formed between the cartridge and the sterility barrier. Preferably, the step of inserting a cartridge into the recess is carried out in a sterile environment. This method can provide a complete sterile environment, not just for the needle but also for the part of the cartridge that the needle pierces. This can reduce the chance of contaminants.

A fifth aspect of the invention concerns a medicament delivery device such as an autoinjector comprising a medicament delivery member housing such as a needle, a medicament delivery member attached to the medicament delivery member housing, the medicament delivery member comprising a proximal end for injection and a distal end for piercing a cartridge, a sterility barrier attached to the medicament delivery member housing, wherein the medicament delivery member housing and the sterility barrier form a first sealed cavity, and the distal end of the medicament delivery member is in the first sealed cavity, and wherein a distal portion of the sterility barrier is configured to form a second sealed cavity between the sterility barrier and a cartridge when a cartridge is inserted into the distal portion of the sterility barrier.

A sixth aspect of the invention concerns a medicament delivery member housing assembly comprising a medicament delivery member housing, a medicament delivery member arranged in the medicament delivery member housing, a sterility barrier attached to the medicament delivery member housing to form a sterile cavity, wherein at least part of the medicament delivery member is in the sterile cavity, wherein the housing is configured to receive a sealed cartridge in a recess formed by the sterility barrier and/or the medicament delivery member housing so as to form a seal between the housing and the cartridge. Preferably, a second sterile cavity is formed between the housing and the cartridge.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the invention will now be described by way of example only and with reference to the accompanying drawings, in which:
Figure 1 shows a perspective view of a needle housing assembly.
Figure 2 shows a cross-sectional view of an autoinjector sub-assembly including a needle housing assembly as shown in Figure 1.
Figure 3 shows an exploded perspective view of the autoinjector sub-assembly of Figure 2.
Figure 4 shows a perspective view of part of the autoinjector sub-assembly of Figure 2.
Figure 5 shows a perspective view of the autoinjector sub-assembly of Figure 2.
Figure 6 shows a perspective view of the sterility barrier of the needle housing assembly of Figure 1.
Figure 7 shows another perspective view of the sterility barrier of the needle housing assembly of Figure 1

### DETAILED DESCRIPTION

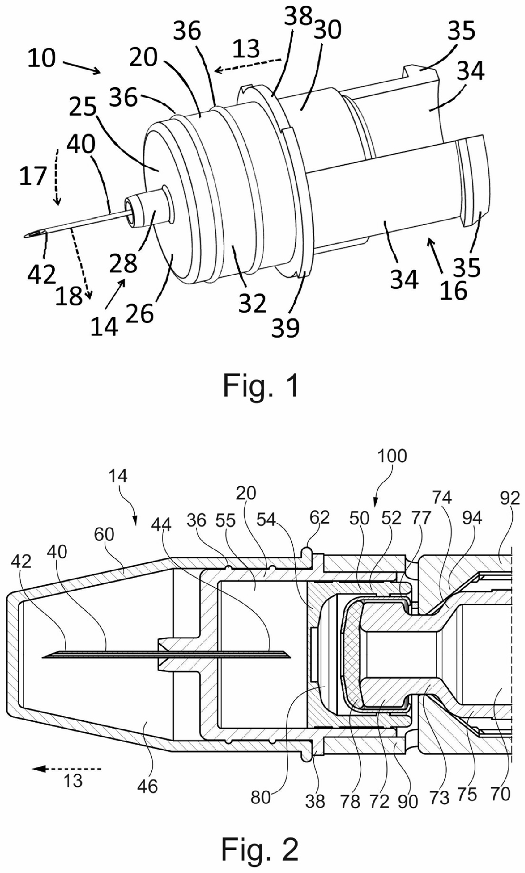

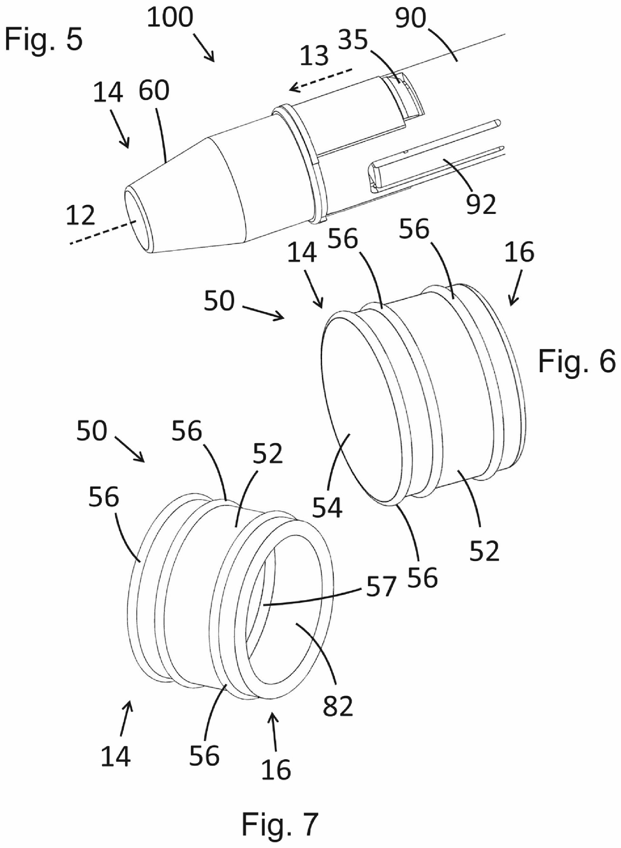

Initially, an example needle housing assembly (in this example an autoinjector needle-housing assembly) will be described. Figure 1 shows a needle housing assembly 10, and Figure 2 shows the needle housing assembly in context in an autoinjector sub-assembly 100. The needle housing assembly 10 comprises a housing 20, a needle 40, and a sterility barrier 50. The housing 20 comprises a proximal part 25 and a distal part 30. The needle 40 is fixedly attached to the proximal part 25 of the housing. The sterility barrier 50 is attached to the distal part 30 of the housing. The housing 20 and the sterility barrier 50 form a sealed cavity 55, wherein a distal end 44 of the needle 40 is in the sealed cavity 55. The needle housing assembly comprises a recess 82 (see Figure 7) formed by the distal end 16 of the needle housing assembly 10, wherein the recess 82 is shaped to receive a cartridge 70 (for example the a head 72 of the cartridge 70) and to form a second sealed cavity 80 between the needle housing assembly 10 and said cartridge 70.

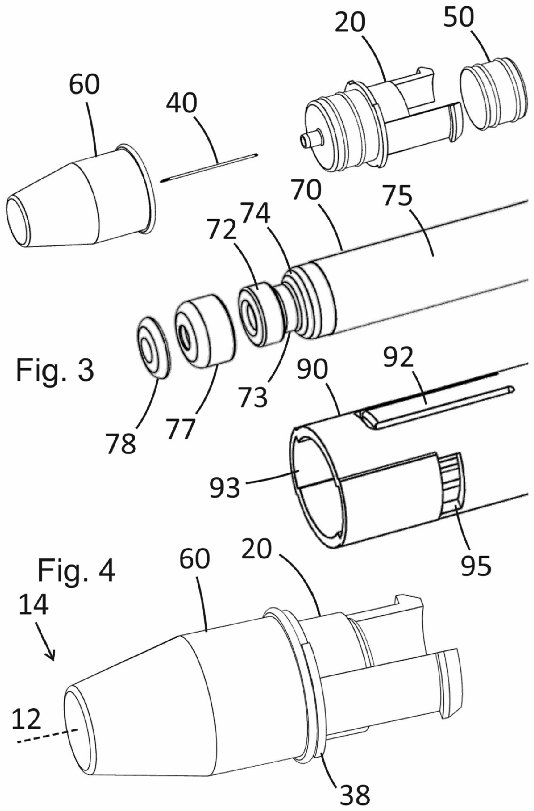

The example shown in the figures will now be described in more detail with references to Figures 1 and 2 in particular. Further detail is also shown in Figures 3, 4 and 5, showing various views of the autoinjector sub-assembly 100, and Figures 6 and 7, which show the sterility barrier more closely. The housing 20 comprises a proximal part 25 and a distal part 30. The distal part 30 comprises a tubular body 32, two optional arms 34 extending in the distal direction from the distal end of the tubular body 32, two optional seals 36 extending around the outside of the tubular body 32, and an optional flange 38 extending around the outside of the tubular body 32. Each arm 34 optionally comprises a protrusion 35 extending radially outwards at the distal end of the arm 34 to engage with a corresponding optional cut-out 95 in the cartridge holder (see Figure 3). The cut-out 95 may extend partially or completely through the cartridge holder in the radial direction.

The proximal part 25 comprises a wall 26 that extends radially from the proximal end of the tubular body 32 and a needle attachment part 28 attached to the wall 26. In this example the needle attachment part 28 is a tubular structure extending in the longitudinal direction from the wall 26, though other shapes are also possible for the needle attachment part 28.

The sterility barrier 50 comprises a tubular body 52 and a wall 54 that extends radially from the proximal end of the tubular body 52, forming a recess 82. As can be seen in more detail in Figures 6 and 7, three seals 56 (outer seals of the sterility barrier) extend around the outside of the tubular body 52. The seals are spaced apart in the longitudinal direction, with the proximal-most seal adjacent to the wall 54. Similarly, a seal 57 (inner seal of the sterility barrier) extends around the inside of the tubular body 52. The tubular body 52 of the sterility barrier is shaped to fit inside the tubular body 32 of the housing so that the tubular body 52 of the sterility barrier is movable in the longitudinal direction relative to the tubular body 32 of the housing 20.

Figures 2 and 3 show the needle housing assembly of Figure 1 in context in an example autoinjector sub-assembly. Along with the housing 20, the needle 40 and the sterility barrier 50, a needle shield 60, a cartridge 70 and a cartridge holder 90 are shown. As can be seen in Figure 2, a distal portion of the needle shield 60 extends around the housing 20 (specifically the tubular body 32 of the housing 20) to create a needle shield cavity 46. An optional flange 62 of the needle shield 60 abuts the flange 38 of the housing 20. Optional seals 36 can help create an airtight seal between the housing 20 and the needle shield 60.

The cartridge 70 comprises a cartridge head 72, a cartridge neck 73, a cartridge shoulder 74 and a cartridge body 75, along with a cartridge crimp 77 and a cartridge septum 78. The cartridge would typically also include a stopper in a distal portion of the cartridge (not shown). The cartridge septum 78 is held in place across a hole in the cartridge by the cartridge crimp 77. The cartridge is filled with a fluid such as a liquid medicament. As described further below, the proximal end of the cartridge 70 forms a cartridge cavity 80 along with the sterility barrier 50. An optional seal 57 can help create an airtight seal between the sterility barrier 50 and the cartridge 70. Typically only a proximal end of the cartridge, in this example the head of the cartridge, is inside the recess 82.

The cartridge holder 90 abuts the flange 38 of the housing 20 at the proximal end of the cartridge holder, and a proximal portion of the cartridge holder extends around a distal portion of the tubular body of the housing 20, as can be seen in Figure 2. This particular design can help align the parts during assembly, although the flange 62 and the flange 38 are both optional. The cartridge holder may comprise arms 92, with the arms 92 comprising protrusions 94 extending towards the axis 12 to support the cartridge 70 (in this example, the shoulder 74 of the cartridge 70). The arms 92 are flexible to allow the protrusions 94 to move out of the way of the cartridge 70 when the cartridge 70 is inserted into the cartridge holder 90 during assembly.

The structure described above results in three sealed cavities, namely the needle shield cavity 46, the housing cavity 55 and the cartridge cavity 80. The needle shield cavity is formed by the needle shield 60 and the proximal part 25 of the housing 20. The housing cavity 55 is formed by the housing 20 (specifically by the tubular body 32 and the wall 26 in this example) and the sterility barrier (specifically by the wall 54 in this example). The cartridge cavity 80 is formed by the sterility barrier 50 (specifically the tubular body 52 and the wall 54 in this example) and the cartridge 70 (specifically the cartridge crimp 77 and the cartridge septum 78 in this example).

During assembly, the needle housing assembly would typically be assembled (preferably in a sterile environment to provide a sterile environment in the housing cavity 55), followed by the addition of the needle shield (again preferably in a sterile environment to provide a sterile environment in the needle shield cavity 46). This would then be followed by the insertion of the cartridge into the needle housing assembly (again preferably in a sterile environment to provide a sterile environment in the cartridge cavity 80). Before or after insertion of the cartridge into the needle housing assembly, the cartridge is inserted into the cartridge holder. The entire assembly process can take place in the same location, but sub-assemblies (for example the sub-assembly shown in Figure 4 consisting of the needle housing assembly and the needle shield) could also be assembled in one location and then transported to another location for further assembly. During insertion of the cartridge into the needle housing assembly, the sterility barrier remains in place relative to the housing (i.e. it does not move relative to the housing). Alternatively, the cartridge can push the sterility barrier in the proximal direction relative to the housing as the cartridge is inserted. If the sterility barrier moves during cartridge insertion, this can optionally result in the distal end of the needle piercing the sterility barrier (specifically the wall 54 of the sterility barrier) during cartridge insertion. In this case, only the cartridge (specifically the septum of the cartridge) needs to be pierced during subsequent device use.

To use an autoinjector comprising the assemblies described herein, for example the assembly shown in Figure 2, the needle shield is first removed (typically at the same time as a corresponding cap). The cartridge is then pushed in the proximal direction by a mechanism within the autoinjector (or directly by the action of a user), which results in the sterility barrier also moving in the proximal direction. As the sterility barrier and the cartridge move forward, first the sterility barrier (specifically the wall 54 of the sterility barrier) and then the septum of the cartridge are pierced by the distal end 44 of the needle. This allows injection of the substance in the cartridge to commence (typically driven by a powerpack within the autoinjector).

The examples given herein primarily relate to autoinjectors. However, other medicament delivery devices such as pen injectors could also include the designs described herein.

The examples given herein include a needle. Whilst this design is primarily for needle-based injection devices, other medicament delivery members, such as a jet injector, could alternatively be used in the designs described herein, for example by replacing the needle or the front portion of the needle (the part in the needle shield cavity) with a jet injector.

The examples described herein would typically be implemented in autoinjectors. In addition to the parts described herein, for example those in the autoinjector sub-assembly 100, an autoinjector would generally comprise an outer housing, a cap, a needle guard, and a powerpack for pushing a stopper of the cartridge towards the proximal end of the autoinjector to expel the medicament from the cartridge. Typically, an autoinjector is tubular in shape.

An autoinjector comprising assemblies as described herein typically comprises an outer housing, a cap, a needle guard, the cartridge holder (which may be part of the outer housing), the needle shield (for example a rigid needle shield and/or a flexible needle shield), the needle, the cartridge, a medicament in the cartridge, and a powerpack.

The housing described above is primarily a wall extending radially (proximal part) attached to a tubular body extending longitudinally (distal part). The shape of these parts may be varied; for example, the tubular body 32 may be a tube of fixed diameter as shown or may have a varying diameter. The tubular body may be a conical or frusto-conical shape, with the diameter at the distal end of the tubular body being larger than the diameter at the proximal end of the tubular body.

In the example shown in the figures, the sterility barrier 50 and the housing 20 are separate components. However, they can also be a single integral part, with the sterility barrier therefore fixed relative to the housing and the needle being moveable relative to the housing instead.

The proximal part 25 of the housing comprises a wall extending in the radial direction to form the proximal end wall of the housing cavity 55 (although the proximal part might be only a small part of the cavity-defining structure in embodiments where the tubular body of the housing is conical or frusto-conical). The proximal part 25 also supports the needle. The distal part 30 of the housing typically comprises most of the structure of the housing, and comprises the tubular body.

The particular structure of the optional arms (distally extending arms 34) and protrusions 35 (radially outwardly extending protrusions 35) is merely exemplary, and could be varied according to the shape of adjacent components. As with many features of autoinjectors, multiple arms are provided (two in this case), each with a single protrusion, but one, three or more arms could alternatively be provided, and multiple protrusions could be provided on each arm, again typically depending on the particular shape of the corresponding adjacent components. The arms are arranged to engage the cartridge holder in the example above, but could alternatively engage a different component such as an outer housing or part of a powerpack.

Several seals 36, 56, 57 are shown in the example described above. Although a particular number of seals is shown in each case in the example described above, one, two or more seals (either identical seals or different seals) can be arranged in any particular gap, depending for example on the quality of the seals used and on how good the seal should be. Where multiple seals are shown in the example described above, the seals are spaced apart in the longitudinal direction, but they could alternatively be adjacent to one another. Optionally, one or more of the seals may be a one-way seal, so that air can escape from a cavity but cannot be let in - this may be beneficial to allow air to escape as cavity sizes change due to components moving relative to one another (e.g. the sterility barrier moving relative to the housing and reducing the volume of the housing cavity).

Each seal 36 can be part of the needle shield, part of the housing, or an independent component such as an O-ring. Each seal 56 can be part of the housing, part of the sterility barrier, or an independent component such as an O-ring. Each seal 57 can be part of the sterility barrier, part of the cartridge, part of the housing, or an independent component such as an O-ring. Instead of providing a seal, an airtight fit could be provided between the respective components in one or more of the three gaps in which seals are shown in the example shown in the Figures. However, the provision of one or more seals may be beneficial as it can allow for broader manufacturing tolerances for the components.

Flange 38 is optional, but the provision of flange 38 may help with assembly, for example by helping to position the needle shield 60 and/or the cartridge holder 90 in the circumferential and/or longitudinal directions. As an example, optional protrusions 39 (see Figure 1) can be provided on the flange to engage corresponding cavities 93 (see Figure 3) on the inside surface of the cartridge holder to help align the parts correctly in the circumferential direction 17.

The needle 40 is typically fixed relative to the housing 20, but could alternatively be moveable in the longitudinal direction relative to the housing 20 as mentioned above. The proximal end 42 of the needle and the distal end 44 of the needle are both shown with angled ends for ease of penetration of the injection site and the cartridge septum respectively; these features are optional. Typically, the distal end 44 of the needle is spaced apart from the sterility barrier 50 (specifically from the wall 54 of the sterility barrier). As a result, the needle is also spaced apart from the cartridge 70 when the needle housing assembly 10 is provided in an autoinjector. Typically, the proximal end 42 of the needle is spaced apart from the needle shield 60 when the needle housing assembly 10 is provided in an autoinjector.

The sterility barrier 50 may be part of the housing 20 or, as shown in the Figures, a separate component. The sterility barrier comprises a wall 54 and can additionally comprise a tubular body 52.

The tubular body 52 of the sterility barrier is shaped to fit inside the tubular body 32 of the housing. As such, the outer diameter of the tubular body 52 of the sterility barrier is smaller than the inner diameter of the tubular body 32 of the housing. The tubular body 52 of the sterility barrier is arranged to move in the longitudinal direction 13 relative to the tubular body 32 of the housing. To enable this, at least a portion of the inner surface of the tubular body 32 of the housing has a constant diameter, and at least a corresponding portion of the outer surface of the tubular body 52 of the housing has a constant diameter (in the example shown, see for example Figure 2, the outer surface of the tubular body 52 of the housing has a constant diameter for its entire length in the longitudinal direction, and the inner surface of the tubular body 32 of the housing has a constant diameter for its entire length in the longitudinal direction). This is not essential, however, as the point at which the sterility barrier moves relative to the housing is typically just before injection, at which point the cavities typically do not need to be kept airtight any more.

The sterility barrier as shown in the Figures comprises a tubular body 52 and a wall 54. In some embodiments, the tubular body 52 is not necessary, with another component (for example the distal part of the tubular body 32 of the housing) helping to form the cartridge cavity 80.

The needle shield 60 may be a rigid needle shield or a flexible needle shield. When then needle shield 60 is a flexible needle shield, a rigid needle shield attached to the flexible needle shield may additionally be provided. The particular shape of the needle shield 60 as shown in the Figures is not essential, and may be varied, for example depending on the shape and size of other components such as the housing 20 or a cap of an autoinjector.

The cartridge 70 shown in the Figures has a cartridge exit hole at the proximal end of the cartridge, along with a particular cartridge shape and a particular septum and crimp design to seal the cartridge exit hole, with an optional hole in the crimp to allow the needle direct access to the septum. The shape and design of the cartridge (including the shape and design of the head, neck, shoulder and body of the cartridge) can be varied, as can the method of closure of the cartridge (a crimp and septum design in this case), and a particular cartridge style, closure, shape or design is not essential to the invention described herein. Typically, the specific design of a needle housing assembly (particularly the distal part 30 of the housing 20, the sterility barrier 50 and/or the needle 40) would be adjusted according to the particular properties of the proposed cartridge.

The cartridge cavity 80 as shown in the Figures is formed by the sterility barrier and the cartridge, and is a result of inserting the cartridge into the recess 82 of the sterility barrier. Alternatively, the cartridge cavity may be formed by the sterility barrier, the housing and the cartridge, depending on the shape of the sterility barrier in particular. In such cases, the recess 82 is typically formed by a combination of the sterility barrier and the housing, for example by a combination of the sterility barrier and the tubular body of the housing.

The cartridge holder as shown herein is optional, and its function (supporting the cartridge) may be provided by other components such as an outer housing. One example of how the cartridge holder can be supported is shown in the Figures, with the arms 92 (in this case two arms) of the cartridge holder (specifically an angled surface of the protrusion 94 on the arm) supporting the shoulder 74 of the cartridge, with the angled surface of the protrusion corresponding to the angle of the shoulder. As with other duplicated features shown herein, one, two, three or more arms could be provided.

Various modifications to the embodiments described are possible and will occur to those skilled in the art without departing from the invention which is defined by the following claims.

## Claims

1. An autoinjector needle-housing assembly (10) extending along an axis (12) in a longitudinal direction (13) from a proximal end (14) to a distal end (16), the autoinjector needle-housing assembly (10) comprising
a housing (20) comprising a proximal part (25) and a distal part (30) attached to the proximal part (25),
a needle (40) fixedly attached to the proximal part (25) of the housing (20), and
a sterility barrier (50) attached to the distal part (30) of the housing (20), wherein the housing (20) and the sterility barrier (50) form a first sealed cavity (55), wherein a distal end (44) of the needle (40) is in the first sealed cavity (55),
wherein the autoinjector needle-housing assembly (10) comprises a recess (82) formed by the distal end of the autoinjector needle-housing assembly (10), wherein the recess (82) is shaped to receive a cartridge (70) and to form a second sealed cavity (80) between the autoinjector needle-housing assembly (10) and said cartridge (70), wherein the sterility barrier (50) comprises a seal (57) on a tubular portion of the sterility barrier (50),
wherein the seal (57) is on the inner surface of the tubular portion, and
wherein the seal (57) helps create an airtight seal between the sterility barrier (50) and the cartridge (70).

2. The autoinjector needle-housing assembly of claim 1, wherein the recess is formed between the sterility barrier of the autoinjector needle-housing assembly and said cartridge.

3. The autoinjector needle-housing assembly of claim 1 or 2, wherein the sterility barrier comprises a tubular portion and a wall extending across the tubular portion, wherein the tubular portion of the sterility barrier extends in the longitudinal direction and the wall extends in a radial direction relative to the axis.

4. The autoinjector needle-housing assembly of claim 3, wherein the sterility barrier comprises a seal on the tubular portion of the sterility barrier.

5. The autoinjector needle-housing assembly of claim 4, wherein the seal is an O-ring.

6. The autoinjector needle-housing assembly of any previous claim, wherein the distal part of the housing comprises a tubular section and the proximal part of the housing comprises a wall extending in a radial direction relative to the axis, the wall extending across the proximal end of the tubular section.

7. The autoinjector needle-housing assembly of a combination of claims 3 and 6, wherein the first sealed cavity is tubular, and is formed by the tubular section of the housing, the wall of the housing and the wall of the sterility barrier.

8. The autoinjector needle-housing assembly of claim 6 or 7, wherein the tubular section of the sterility barrier is in the tubular section of the housing.

9. The autoinjector needle-housing assembly of any previous claim,
wherein the sterility barrier is movable in the longitudinal direction relative to the housing.

10. An autoinjector sub-assembly comprising the autoinjector needle-housing assembly of any previous claim and a needle shield attached to the autoinjector needle-housing assembly,
wherein the needle shield and the autoinjector needle-housing assembly define a third sealed cavity, and wherein a proximal end of the needle is in the third sealed cavity.

11. An autoinjector comprising a cartridge and either the autoinjector needle-housing assembly of any of claims 1 to 9 or the autoinjector sub-assembly of claim 10, wherein a proximal end of the cartridge is in the distal recess of the autoinjector needle-housing assembly.
